# EUROPÄISCHE PATENTSCHRIFT

(11) **EP 2 389 402 B1**
(45) Veröffentlichungstag und Bekanntmachung des Hinweises auf die Patenterteilung: **20.03.2013**
(21) Anmeldenummer: 10700703.1
(22) Anmeldetag: 13.01.2010
(51) Int. Cl.: C08G 18/10, C08G 18/12, C08G 18/08, C08G 18/28, C08G 18/48, C08G 18/72, C08G 18/73, C08G 18/75, A61L 15/48, A61L 15/26, A61L 15/42, C08J 9/00

(54) **Polyurethan-Tensid-stabilisierte Polyurethan-Schäume**
Polyurethane-tenside stabilised polyurethane foams
Mousses de polyuréthanes stabilisées par tensioactif à base de polyuréthane

(30) Priorität: 24.01.2009 EP 09000993
(43) Veröffentlichungstag der Anmeldung: 30.11.2011
(73) Patentinhaber: Bayer MaterialScience AG, 51368 Leverkusen (DE)
(72) Erfinder: SCHÖNBERGER, Jan, 42781 Haan (DE); DÖRR, Sebastian, 40593 Düsseldorf (DE)
(86) Internationale Anmeldenummer: PCT/EP2010/000120
(87) Internationale Veröffentlichungsnummer: WO 2010/083952

(56) Entgegenhaltungen:
- EP-A- 0 495 373
- WO-A-96/34904
- WO-A-2007/115697
- US-A1- 2002 042 492

## Beschreibung

Die Erfindung betrifft die Herstellung von hydrophilierten Polyurethan-Schäumen, insbesondere für die Wundbehandlung, bei welcher eine Zusammensetzung, enthaltend ein Polymer und spezielle polyurethanbasierte Stabilisatoren, aufgeschäumt und getrocknet wird.

Die Verwendung von Wundauflagen aus Schäumen zur Behandlung von nässenden Wunden ist Stand der Technik. Aufgrund ihres hohen Absorptionsvermögens und ihrer guten mechanischen Eigenschaften werden in der Regel Polyurethan-Schäume eingesetzt, welche durch Umsetzung von Mischungen aus Diisocyanaten und Polyolen bzw. NCO-funktionellen Polyurethan-Prepolymeren mit Wasser in Gegenwart bestimmter Katalysatoren sowie (Schaum-) Additiven hergestellt werden. In der Regel werden hierbei aromatische Diisocyanate eingesetzt, da sich diese am besten Verschäumen lassen. Zahlreiche Ausführungsformen dieser Verfahren sind bekannt, beispielsweise beschrieben in US 3,978,266, US 3,975,567 und EP-A 0 059 048. Die vorgenannten Verfahren weisen jedoch den Nachteil auf, dass reaktive Mischungen eingesetzt werden müssen, enthaltend Diisocyanate oder entsprechende NCO-funktionelle Prepolymere, deren Handhabung technisch aufwendig ist, da z.B. entsprechende Schutzmaßnahmen erforderlich sind.

Eine Alternative zum vorstehend beschriebenen Verfahren, in welchem Diisocyanate bzw. NCO-funktionelle Polyurethan-Prepolymere eingesetzt werden, ist ein Verfahren basierend auf Polyurethan-Dispersionen (die im Wesentlichen frei von Isocyanatgruppen sind), in welche man in Gegenwart geeigneter (Schaum-) Additive durch kräftiges Rühren Luft oder andere Gase einträgt. Nach dem Trocknen und Aushärten werden so genannte mechanische Polyurethan-Schäume erhalten. Solche Schäume sind im Zusammenhang mit Wundauflagen in EP-A 0 235 949 und EP-A 0 246 723 beschrieben, wobei dem Schaum entweder ein selbsthaftendes Polymer zugesetzt oder der Schaum auf einen Film eines selbsthaftenden Polymers aufgebracht wird. In US 4,655,210 ist die Verwendung der vorgenannten mechanischen Schäume für Wundauflagen beschrieben, die einen speziellen Aufbau aus Träger, Schaum und Hautkontakt-Schicht aufweisen. Wie in EP-A 0 235 949, EP-A 0 246 723 und US 4,655,210 beschrieben, wurden zur Herstellung der Schäume aus den Polyurethan-Dispersionen stets Additiv-Mischungen eingesetzt, welche Ammoniumstearat enthalten. Dies ist jedoch von großem Nachteil, da Ammoniumstearat in den üblicherweise eingesetzten Mengen zu stark zytotoxischen Schäumen führt. Dies ist insbesondere bei Wundauflage-Schäumen nicht akzeptabel. Darüber hinaus ist Ammoniumstearat thermisch zersetzlich, und der gebildete Ammoniak muss mit entsprechendem technischen Aufwand entsorgt werden. Andererseits hat sich gezeigt, dass Ammoniumstearat nicht einfach durch andere Stearate oder gänzlich andere (Schaum-) Additive ersetzt werden kann, da keine vergleichbar gute Schaumstruktur, gekennzeichnet vor allem durch sehr feine Poren, erhalten wird.

Eine alternative Stabilisierung von PUR-Schäumen mittels Alkylpolyglykosiden ist in WO 2008/031520 beschrieben. Diese Additive zeigen jedoch ebenfalls eine stark zytotoxische Wirkung und verschlechtern zudem die Porosität der erhaltenen Schäume merklich. Des weiteren verursachen diese Schaumadditive eine unerwünschte Vergilbung der Schäume, was möglicherweise auf eine Verstoffwechselung dieser Glykoside durch Mikroorganismen zurückzuführen ist.

EP 0731148 beschreibt hydrophil modifizierte, verzweigte Polyisocyanataddukte basierend auf Polyisocyanaten mit einer mittleren NCO-Funktionalität von mindestens 2,5, welche mit hydrophilen Polyethern umgesetzt werden. Diese Komponenten haben den Nachteil, dass durch den relativ hohen Grad an Verzweigung keine optimale Entfaltung des hydrophilen Potentials der Polyetherkette möglich ist, da aus sterischen Gründen mehr als 2 Polyetherketten nicht gleichzeitig vollständig in der Wasserphase sein können, wenn der hydrophobe Rest des Adduktes gleichzeitig an einer hydrophoben Phase lokalisiert ist. Dadurch findet sich bei den in EP 0731148 beschriebenen Dispergierhilfsmitteln immer ein Teil des hydrophilen Rests in der Nähe der hydrophoben Phase.

Aufgabe der vorliegenden Erfindung war daher die Bereitstellung von geeigneten Tensiden als (Schaum-) Additive, welche in Kombination mit wässrigen Polyurethandispersionen aufgeschäumt werden können und nach Trocknung feinporige und auch in großen Schichtdicken homogene Schäume liefern, welche nicht zytotoxisch und weitestgehend frei von (thermisch) abspaltbaren Komponenten wie Aminen sind.

Es wurde nun gefunden, dass die zugrunde liegende Aufgabe durch neue Tenside auf Basis von Polyurethanen als Additiv gelöst werden kann.

Gegenstand der Erfindung ist daher ein Verfahren zur Herstellung von Polymerschäumen, bei dem eine Zusammensetzung, welche ebenfalls Gegenstand der Erfindung ist, die erhältlich ist durch Mischen von wenigstens Polyurethanen (I) mit einem Gehalt an freien Isocyanatgruppen von max. 1,0 Gew.-% und einem Gehalt an über monofunktionelle Alkohole B) eingebauten, innerhalb von Polyetherketten angeordneten Ethylenoxideinheiten (Molekulargewicht = 44 g/mol) von 10 bis 95 Gew.-%, die durch Umsetzung von
A) Polyisocyanat-Prepolymeren mit einer (mittleren) NCO-Funktionalität von größer oder gleich 1, bevorzugt 1,7 bis 2,5, besonders bevorzugt 1,8 bis 2,2, ganz besonders bevorzugt 2 mit
B) 10 bis 100 Äquivalent-%, bezogen auf die Isocyanatgruppen von A), einer einwertigen Alkoholkomponente, umfassend mindestens einen einwertigen Polyetheralkohol mit einem zahlenmittleren Molekulargewicht von 150 bis 5000 g/mol mit einem Gehalt an Oxyethyleneinheiten von 30 bis 100 Mol.-% bezogen auf den Gesamtgehalt an Oxyalkyleneinheiten des einwertigen Polyetheralkohols,
C) 0 bis 20 Äquivalent-%, bezogen auf die Isocyanatgruppen von A), einer einwertigen Alkoholkomponente, umfassend von der Verbindungen der Komponente B) verschiedene einwertige Alkohole mit einem zahlenmittleren Molekulargewicht von 32 bis 5000 g/mol,
D) 0 bis 80 Äquivalent-%, bezogen auf die Isocyanatgruppen von A), an im Sinne der NCO-Additionsreaktion mindestens difunktionellen Aufbaukomponenten mit einem zahlenmittleren Molekulargewicht von 32 bis 10000 g/mol
unter Urethan- und gegebenenfalls Hamstoffbildung hergestellt worden sind, wobei gegebenenfalls im Überschuss vorliegende NCO-Gruppen durch gleichzeitig oder anschließend erfolgende Sekundärreaktionen bis auf einen Restgehalt von maximal 1,0 Gew.-% abreagiert worden sind
und schäumbaren Polymeren (II), aufgeschäumt und getrocknet wird.

In den erfindungswesentlichen Polyurethanen beträgt der Gehalt an Ethylenoxideinheiten (Molekulargewicht = 47 g/mol) bevorzugt 20 bis 75 Gew.-%, besonders bevorzugt 35 bis 60 Gew.-% und ganz besonders bevorzugt 45 bis 55 Gew.%. Der Gehalt an freien Isocyanatgruppen in den erfindungswesentlichen Polyurethanen liegt unter 1 Gew.-%, im Allgemeinen sind freie Isocyanatgruppen nicht mehr nachweisbar.

Geeignete Polyisocyanat-Prepolymere der Komponente A) sind die dem Fachmann an sich bekannten aliphatischen, aromatischen oder cycloaliphatischen isocyanatfunktionelle Prepolymere mit den vorgenannten NCO-Funktionalitäten.

Die in A) einsetzbaren isocyanatfunktionellen Prepolymere sind durch Umsetzung von Polyisocyanaten mit hydroxyfunktionellen Polyolen gegebenenfalls unter Zusatz von Katalysatoren sowie Hilfs- und Zusatzstoffen erhältlich.

Beispiele solcher geeigneten isocyanatfunktionelle Bausteine A) sind Prepolymere basierend auf Polyolen und niedermolekularen Isocyanat-Bausteinen. Niedermolekulare Isocyanat-Bausteine sind Verbindungen wie 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts, 1,4-Cyclohexylendiisocyanat, 4-Isocyanatomethyl-1,8-octandiisocyanat (Nonantriisocyanat), 1,4-Phenylendiisocyanat, 2,4- und/oder 2,6- Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysindiisocyanate) mit C1-C8-Alkylgruppen.

Die isocyanatfunktionellen Bausteine A) können beispielsweise Uretdion-, Isocyanurat-, Urethan-, harnstoff-, Allophanat-, Biuret-, Iminooxadiazindion- oder Oxadiazintrionstrukturen sowie Mischungen aus diesen enthalten.

Die polymeren Polyole zur Herstellung von A) sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können zur Herstellung des Prepolymers A) einzeln oder in beliebigen Mischungen untereinander eingesetzt werden.

Geeignete Polyesterpolyole sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,6-Hexandiol und Isomere, Neopentylglykol oder Hydroxypivalinsäureneopentylglykolester, wobei 1,6-Hexandiol und Isomere, 1,4-Butandiol, Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbemsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Auch niedermolekulare Polyole können zur Herstellung von A) eingesetzt werden. Beispiele derartiger Polyole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Der Einsatz von Polyetherpolyole zur Herstellung von A) ist bevorzugt.

Die Polyetherpolyole zur Herstellung der Komponente A) haben in der Regel zahlenmittlere Molekulargewichte Mn von 300 bis 8000 g/mol, bevorzugt 400 bis 6000 g/mol, besonders bevorzugt 600 bis 3000 g/mol.

Ferner weisen sie besonders bevorzugt einen Gehalt an ungesättigten Endgruppen von kleiner oder gleich 0,02 Milliäquivalenten pro Gramm Polyol (meq/g), bevorzugt kleiner oder gleich 0,015 meq/g, besonders bevorzugt kleiner oder gleich 0,01 meq/g (Bestimmungsmethode ASTM D2849-69) auf.

Die zur Herstellung der Verbindungen der Komponente A) eingesetzten Polyole weisen bevorzugt eine OH-Funktionalität von 1,5 bis 4, besonders bevorzugt von 1,8 bis 2,5, ganz besonders bevorzugt von 1,9 bis 2,1 auf.

Besonders bevorzugt haben sie eine besonders enge Molekulargewichtsverteilung, d.h. eine Polydispersität (PD = Mw/Mn) von 1,0 bis 1,5 und/oder eine OH-Funktionalität von größer 1,9. Bevorzugt weisen die genannten Polyetherpolyole eine Polydispersität von 1,0 bis 1,5 und eine OH-Funktionalität von größer 1,9 auf, besonders bevorzugt größer oder gleich 1,95 auf.

Solche Polyetherpolyole sind in an sich bekannter Weise durch Alkoxylierung von geeigneten Starter-Molekülen, insbesondere unter Verwendung von Doppelmetallcyanid-Katalysatoren (DMC-Katalyse) herstellbar. Dies ist z.B. in der US-A 5158 922 (z.B. Beispiel 30) und EP-A 0 654 302 (S. 5, Z. 26 bis S. 6, Z. 32) beschrieben.

Geeignete Starter-Moleküle für die Herstellung der Polyetherpolyole sind beispielsweise einfache, niedermolekulare Polyole, Wasser, organische Polyamine mit mindestens zwei N-H-Bindungen oder beliebige Gemische derartiger Starter-Moleküle. Für die Alkoxylierung geeignete Alkylenoxide sind insbesondere Ethylenoxid und/oder Propylenoxid, die in beliebiger Reihenfolge oder auch im Gemisch bei der Alkoxylierung eingesetzt werden können.

Bevorzugte Starter-Moleküle zur Herstellung der Polyetherpolyole durch Alkoxylierung, insbesondere nach dem DMC-Verfahren, sind insbesondere einfache Polyole wie Ethylenglykol, Diethylenglykol, Triethylenglykol, Butyldiglykol, 1,3-Butylenglykol, 1,3-Propylenglykol, 1,2-Propandiol, 1,3-Propandiol, 1,4-Butandiol, 1,6-Hexandiol, Cyclohexandiol, 1,4-Cyclohexandimethanol, Neopentylglykol, 2-Ethylhexandiol-1,3, Glyzerin, Trimethylolethan, Trimethylolpropan, Pentaerythrit, Sorbit, Triethanolamin, Hydrochinondihydroxyethylether, Bisphenol A (2,2-Bis(4-hydroxyphenyl)propan), hydriertes Bisphenol A (2,2-Bis(4-hydroxycyclohexyl)propan) sowie niedermolekulare, Hydroxylgruppen aufweisende Ester derartiger Polyole mit Dicarbonsäuren der nachstehend beispielhaft genannten Art oder niedermolekulare Ethoxylierungs- oder Propoxylierungsprodukte derartiger einfacher Polyole oder beliebige Gemische derartiger modifizierter oder nicht modifizierter Alkohole.

Geeignet sind beispielsweise die in der Polyurethanchemie an sich bekannten Polytetramethylenglykolpolyether, wie sie durch Polymerisation von Tetrahydrofuran mittels kationischer Ringöffnung erhältlich sind, sowie Polypropylenglykol und Polycarbonat-Polyole bzw. deren Mischungen und besonders bevorzugt ist Polypropylenglykol.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle.

Geeignet sind auch Esterdiole des genannten Molekulargewichtsbereichs wie α-Hydroxybutyl-ε-hydroxy-capronsäureester, ω-Hydroxyhexyl-y-hydroxybuttersäureester, Adipinsäure-(β-hydroxyethyl)ester oder Terephthalsäurebis(ß-hydroxyethyl)ester.

Ferner können in auch monofunktionelle isocyanatreaktive Hydroxyl-gruppenhaltige Verbindungen eingesetzt werden. Beispiele solcher monofunktionellen Verbindungen sind Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Weiterhin können NH₂- und/oder NH-funktionelle Komponenten zur Herstellung der Isocyanatprepolymere eingesetzt werden.

Geeignete Komponenten zur Kettenverlängerung sind organische Di- oder Polyamine wie beispielsweise Ethylendiamin, 1,2- und 1,3-Diaminopropan, 1,4-Diaminobutan, 1,6-Diaminohexan, Isophorondiamin, Isomerengemische von 2,2,4- und 2,4,4-Trimethylhexamethylendiamin, 2-Methylpentamethylendiamin, Diethylentriamin, Diaminodicyclohexylmethan und/oder Dimethylethylendiamin.

Darüber hinaus können auch Verbindungen die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-methylaminobutan, Alkanolamine wie N-Aminoethylethanolamin, Ethanolamin, 3-Aminopropanol, Neopentanolamin, welche zur Kettenverlängerung bzw. -terminierung eingesetzt werden. Zur Kettenterminierung werden üblicherweise Amine mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primäre/tertiäre Amine, wie N,N-Dimethylaminopropylamin verwendet.

Bevorzugt handelt es sich bei den Verbindungen der Komponente A) um Prepolymere der vorstehend genannten Art mit ausschließlich aliphatisch oder cycloaliphatisch gebundenen Isocyanatgruppen oder Mischungen aus diesen und einer mittleren NCO-Funktionalität der Mischung von 1,7 bis 2,5; bevorzugt 1,8 bis 2,2; besonders bevorzugt 2.

Besonders bevorzugt werden in A) Polyisocyanat-Prepolymere der vorstehend genannten Art auf Basis von Hexamethylendiisocyanat, Isophorondiisocyanat oder den isomeren Bis-(4,4'-isocyanatocyclohexyl)methanen sowie Mischungen der vorgenannten Diisocyanate eingesetzt.

Zur Herstellung der isocyanatfunktionellen Prepolymere A) werden die niedermolekularen Polyisocyanate mit den Polyolen bei einem NCO/OH-Verhältnis von bevorzugt 2:1 bis 20:1 umgesetzt. Die Reaktionstemperatur beträgt dabei in der Regel 20 bis 160°C, bevorzugt 60 bis 100°C. In einer besonders bevorzugten Ausführungsform wird anschließend der Anteil an nicht umgesetzten Polyisocyanaten mittels geeigneter Methoden abgetrennt. Üblicherweise wird hierfür die Dünnschichtdestillation verwendet, wobei restmonomerenarme Produkte mit Restmonomergehalten von weniger als 5 Gew.-%, bevorzugt weniger als 0,5 Gew.-%, ganz besonders bevorzugt weniger als 0,1 Gew.-% erhalten werden

Geeignete nichtionisch hydrophilierende Verbindungen der Komponente B) sind monofunktionelle Polyoxyalkylenether, die mindestens eine Hydroxygruppe enthalten. Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 30 bis 100 mol% Ethylenoxid- und 0 bis 70 mol-% Propylenoxideinheiten bezogen auf die Gesamtmenge an Oxyalkyleneineiten aufweisen.

Geeignete Startermoleküle für solche Bausteine sind gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Geeignete Bausteine der Komponente C) sind einwertigen Alkoholkomponenten, bestehend aus mindestens einem, von den Alkoholen der Komponente B) verschiedenen, einwertigen Alkohol des zahlenmittleren Molekulargewichtsbereichs 32 bis 5000 g/mol. Beispiele sind Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, Fettalkohole, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, araliphatische Alkohole wie Benzylalkohol, Anisalkohol oder Zimtalkohol.

Auch monofunktionelle Polymere sind einsetzbar wie beispielsweise Polyoxyalkylenether, die eine Hydroxygruppe enthalten und weniger als 30 mol-% Ethylenoxid. Bevorzugt sind monofunktionelle Polypropylenoxidpolyether, die keinerlei Ethylenoxid-Bausteine aufweisen.

Geeignete Bausteine der Komponente D) sind im Sinne der NCO-Additionsreaktion mehrwertige isocyanatreaktive Komponenten des zahlenmittleren Molekulargewichtsbereichs 32 bis 10000 g/mol. Beispiele für niedermolekulare insbesondere Polyole, bevorzugt mit bis zu 20 Kohlenstoffatomen, sind Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)-propan), hydriertes Bisphenol A (2,2-Bis(4-hydroxycyclohexyl)propan), Trimethylolpropan, Glycerin, Pentaerythrit sowie deren beliebige Mischungen untereinander. Auch eingesetzt werden können Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole mit einem zahlenmittleren Molekulargewicht bis 10000 g/mol. Es können auch insbesondere Di- oder Polyamine eingesetzt werden wie 1,2-Ethylendiamin, 1,2- und 1,3-Diaminopropan, 1,4-Diaminobutan, 1,6-Diaminohexan, Isophorondiamin, Isomerengemische von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin. Ebenfalls möglich ist die Verwendung von Hydrazin sowie Hydraziden wie Adipinsäuredihydrazid. Bevorzugt sind Isophorondiamin, 1,2-Ethylendiamin, 1,4-Diaminobutan, und Diethylentriamin. Darüber hinaus können als Komponente D) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-Methylaminobutan, Alkanolamine wie N-Aminoethylethanolamin, Ethanolamin, 3-Aminopropanol, Neopentanolamin. Auch Mischungen der genannten Komponenten sind als Baustein D) einsetzbar.

In einer bevorzugten Variante wird keine Komponente D) eingesetzt, in einer anderen bevorzugten Variante wird als Komponente D) zumindest ein Polyoxyalkylenether eingesetzt. In einer ganz besonders bevorzugten Variante wird als Komponente D) ein Polyoxyalkylenether eingesetzt der zumindest zwei isocyanatreaktive Gruppen wie Hydroxylgruppen enthält und darüber hinaus mindestens 30 mol-%, bezogen auf alle enthaltenen Alkylenoxideinheiten an Ethylenoxideinheiten. Besonders bevorzugt als D) sind difunktionelle Polyalkylenoxidpolyether, die 30 bis 100 mol-% Ethylenoxid- und 0 bis 70 mol-% Propylenoxideinheiten aufweisen und noch bevorzugter sind 70 bis 100 mol-% Ethylenoxid- und 0 bis 30 mol-% Propylenoxideinheit enthalten. Am meisten bevorzugt ist in D) als Alkylenoxideinheiten ausschließlich Ethylenoxid enthalten. Derartige Polyoxyalkylenether sind in an sich bekannter Weise durch Alkoxylierung geeigneter, mindestens difunktioneller Startermoleküle herstellbar.

In einer bevorzugten Variante zur Herstellung der erfindungsgemäßen Polyurethane wird zunächst das isocyanatfunktionelles Prepolymer A) hergestellt, indem ein Diisocyanat der vorstehend genannten Art mit einem Unterschuss an einem hydrophoben Diols wie beispielsweise Polypropylenglykol mit einer zahlenmittleren Molmasse von beispielsweise 2000 g/mol umgesetzt wird. Hierbei liegt das Molverhältnis zwischen Isocyanatgruppen und isocyanatreaktiven Gruppen bevorzugt im Bereich von 2 zu 1 bis 20 zu 1, besonders bevorzugt 5 zu 1 bis 15 zu 1. Nach der Umsetzung der isocyanatreaktiven Gruppen wird in einer bevorzugten Variante zumindest ein Grossteil des verbleibenden Diisocyanates destillativ abgetrennt, beispielsweise über Dünnschichtverdampfer unter Erwärmen im Vakuum. Das dabei erhaltene Gemisch wird anschließend mit Komponente B) und optional Komponente C) und/oder optional mit Komponente D) umgesetzt. Bevorzugt wird als Komponente B) dann ein einwertiger Polyetheralkohol des zahlenmittleren Molekulargewichtsbereichs 350 bis 3000 g/mol, besonders bevorzugt 700 bis 2300 g/mol mit einem bevorzugten Gehalt an Ethylenoxideinheiten von 70 bis 100 Gew.-% bezogen auf den Gesamtgehalt an Oxyalkyleneinheiten eingesetzt.

Das Molverhältnis zwischen Isocyanatgruppen und isocyanatreaktiven Gruppen liegt bei der Reaktion von A) mit B) und optional C) und/oder D) bevorzugt im Bereich von 0,5 zu 1 bis 2 zu 1, besonders bevorzugt 0,7 zu 1 bis 1,2 zu 1 und ganz besonders bevorzugt bei 1 zu 1. Bevorzugter Temperaturbereich für die Umsetzung ist 20 bis 180°C, besonders bevorzugt 40 bis 130°C. Die Reaktion wird bevorzugt so geführt, bis keinerlei Isocyanatgruppen IR-spektroskopisch mehr nachweisbar sind. In einer besonders bevorzugten Variante wird weder C) noch D) eingesetzt, in einer anderen besonders bevorzugten Variante wird ausschließlich D) eingesetzt.

Der Einsatz von dem Fachmann bekannten Katalysatoren ist sowohl bei der Herstellung der Prepolymere A) als auch bei der Herstellung der erfindungsgemäßen Polyurethane möglich. Es können beispielsweise tertiäre Amine, Zinn-, Zink- oder Wismutverbindungen wie Triethylamin, 1,4-Diazabicyclo-[2,2,2]-octan, Zinndioktoat, Dibutylzinndilaurat und Zinkdioktoat zugesetzt werden. Gegebenenfalls können während und/oder nach der Herstellung Stabilisatoren wie Benzoylchlorid, Isophthaloylchlorid, Dibutylphosphat, 3-Chlorpropionsäure, Antioxidantion oder Methyltosylat zugesetzt werden.

Bei der Herstellung der erfindungsgemäßen Polyurethane werden die Komponenten A) bis D) bevorzugt in den nachstehenden Mengenbereichen eingesetzt:
10 bis 80 Gew.%, besonders bevorzugt 20 bis 50 Gew.% Komponente A),
20 bis 90 Gew.%, besonders bevorzugt 30 bis 50 Gew.% Komponente B),
0 bis 15 Gew.%, besonders bevorzugt 0 bis 5 Gew.% Komponente C) und
0 bis 60 Gew.%, besonders bevorzugt 10 bis 30 Gew.% Komponente D).

Neben den genannten Komponenten A), B), C) und D) können noch weitere Isocyanat-Bausteine bzw. Isocyanat-reaktive Bausteine, die nicht unter A), B), C) oder D) fallen, in die erfindungsgemäßen Polyurethane mit eingebaut sein, bevorzugt jedoch mit weniger als 20 Gew.% und ganz besonders bevorzugt sind keine Bausteine außer A), B), C) und D enthalten.

Grundlage für die verwendeten Polymere (II) sind anionisch hydrophilierte Polyurethan- Dispersionen (II), die erhältlich sind, indem
E) isocyanatfunktionelle Prepolymere mindestens aus
   E1) organischen Polyisocyanaten
   E2) polymeren Polyolen mit zahlenmittleren Molekulargewichten von 400 bis 8000 g/mol und OH-Funktionalitäten von 1,5 bis 6 und
   E3) gegebenenfalls hydroxyfunktionellen Verbindungen mit Molekulargewichten von 62 bis 399 g/mol und
   E4) gegebenenfalls isocyanatreaktiven, anionischen oder potentiell anionischen und/oder gegebenenfalls nichtionischen Hydrophilierungsmitteln,
   hergestellt werden,
F) deren freie NCO-Gruppen dann ganz oder teilweise
   F1) gegebenenfalls mit aminofunktionellen Verbindungen mit Molekulargewichten von 32 bis 400 g/mol und/oder
   F2) isocyanatreaktiven, bevorzugt aminofunktionellen, anionischen oder potentiell anionischen Hydrophilierungsmitteln
unter Kettenverlängerung umgesetzt werden und die Prepolymere vor während oder nach Schritt F) in Wasser dispergiert werden, wobei gegebenenfalls enthaltene potentiell ionische Gruppen durch teilweise oder vollständige Umsetzung mit einem Neutralisationsmittel in die ionische Form überführt werden.

Bedeutsam dabei ist, dass die Verbindungen der Komponenten E1) bis E4) keine primären oder sekundären Aminogruppen aufweisen.

Um eine anionische Hydrophilierung zu erreichen, müssen in E4) und/oder F2) Hydrophilierungsmittel eingesetzt werden, die wenigstens eine gegenüber NCO-Gruppen reaktive Gruppe wie Amino-, Hydroxy- oder Thiolgruppen aufweisen und darüber hinaus -COO⁻ oder -SO₃- oder -PO₃²⁻ als anionische bzw. deren ganz oder teilweise protonierte Säureformen als potentiell anionische Gruppen aufweisen.

Bevorzugte wässrige, anionische Polyurethan-Dispersionen (II) haben einen niedrigen Grad an hydrophilen anionischen Gruppen, bevorzugt von 0,1 bis 15 Milliäquivalenten pro 100 g Festharz.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 550 nm, bestimmt mittels Laserkorrelations-Spektroskopie.

Das Verhältnis von NCO-Gruppen der Verbindungen aus Komponente E1) zu NCO-reaktiven Gruppen wie Amino-, Hydroxy- oder Thiolgruppen der Verbindungen der Komponenten E2) bis E4) beträgt bei der Herstellung des NCO-funktionellen Prepolymers 1,05 bis 3,5, bevorzugt 1,2 bis 3,0 besonders bevorzugt 1,3 bis 2,5.

Die aminofunktionellen Verbindungen in Stufe F) werden in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt zwischen 50 bis 125 %, besonders bevorzugt zwischen 60 bis 120 % beträgt.

Geeignete Polyisocyanate der Komponente E1) sind die dem Fachmann an sich bekannten aromatischen, araliphatischen, aliphatischen oder cycloaliphatischen Polyisocyanate einer NCO-Funktionalität von ≥2 und entsprechen denen der Komponente A).

Neben den vorstehend genannten Polyisocyanaten können anteilig auch modifizierte Diisocyanate oder Triisocyanate mit Uretdion-, Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in E1) 1,6-Hexamethylendiisocyanat, Isophorondiisocyanat, die isomeren Bis(4,4`-isocyanatocyclohexyl)methane, sowie deren Mischungen eingesetzt.

In E2) werden polymere Polyole mit einem zahlenmittleren Molekulargewicht Mₙ bevorzugt von 400 bis 6000 g/mol und besonders bevorzugt von 600 bis 3000 g/mol eingesetzt. Diese weisen bevorzugt eine OH-Funktionalität von 1,8 bis 3, besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in E2) einzeln oder in beliebigen Mischungen untereinander eingesetzt werden.

Solche Polyesterpolyole sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbemsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols größer als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in E2) Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.-% Hexandiol bevorzugt 1,6-Hexandiol und/oder Hexandiolderivate bezogen auf die zugrunde liegenden Diole. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in E2) eingesetzt werden.

Die Hydroxylgruppen aufweisenden Polycarbonate sind bevorzugt linear gebaut.

Ebenfalls können in E2) Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrin an di- oder polyfunktionelle Startermoleküle.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan-Dispersionen (II) enthalten als Komponente E2) eine Mischung aus Polycarbonatpolyolen und Polytetramethylenglykolpolyolen, wobei in dieser Mischung der Anteil an Polycarbonatpolyolen in der Mischung 20 bis 80 Gew.-% und der Anteil an Polytetramethylenglykolpolyolen 80 bis 20 Gew.-% beträgt. Bevorzugt ist ein Anteil von 30 bis 75 Gew.-% an Polytetramethylenglykolpolyolen und ein Anteil von 25 bis 70 Gew.-% an Polycarbonatpolyolen. Besonders bevorzugt ist ein Anteil von 35 bis 70 Gew.-% an Polytetramethylenglykolpolyolen und ein Anteil von 30 bis 65 Gew.-% an Polycarbonatpolyolen, jeweils mit der Maßgabe, dass die Summe der Gewichtsprozente der Polycarbonat- und Polytetramethylenglykolpolyole 100% ergibt und der Anteil der Summe der Polycarbonat- und Polytetramethylenglykolpolyetherpolyole an der Komponente E2) mindestens 50 Gew.-%, bevorzugt 60 Gew.-% und besonders bevorzugt mindestens 70 Gew.-% beträgt.

In E3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-ε-hydroxycapronsäureester, ω-Hydroxyhexyl-y-hydroxybuttersäureester, Adipinsäure-(ß-hydroxyethyl)ester oder Terephthalsäurebis(ß-hydroxyethyl)ester.

Ferner können in E3) auch monofunktionelle, hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Ethylen-glykolmonobutylether, Diethylenglykolmonobutylether, Propylenglykolmonomethylether, Dipropylenglykol-monomethylether, Tripropylenglykolmonomethylether, Dipropylenglykol-monopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Bevorzugte Verbindungen der Komponente E3) sind 1,6-Hexandiol. 1,4-Butandiol, Neopentylglykol und Trimethylolpropan.

Unter anionisch bzw. potentiell anionisch hydrophilierenden Verbindungen der Komponente E4) werden sämtliche Verbindungen verstanden, die mindestens eine isocyanatreaktive Gruppe wie eine Hydroxylgruppe aufweisen sowie mindestens eine Funktionalität wie z.B. -COO⁻M⁺,⁻ -SO⁻₃ M⁺, -PO(O⁻M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5-9, Formel I-III beschrieben ist. Bevorzugte anionische oder potentiell anionische Hydrophilierungsmittel der Komponente E4) sind solche der vorstehend genannten Art, die über Carboxylat- bzw. Carbonsäuregruppen und/oder Sulfonatgruppen verfügen.

Besonders bevorzugte anionische oder potentiell anionische Hydrophilierungsmittel der Komponente E4) sind solche, die Carboxylat- bzw. Carbonsäuregruppen als ionische oder potentiell ionische Gruppen enthalten, wie Dimethylolpropionsäure, Dimethylolbuttersäue und Hydroxypivalinsäure bzw. deren Salze.

Geeignete nichtionisch hydrophilierende Verbindungen der Komponente E4) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38).

Diese sind entweder reine Polyethylenoxidether oder gemischte Polyalkylenoxidether, wobei sie dann aber mindestens 30 mol%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente F1) können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin oder sowie Hydraziden wie Adipinsäuredihydrazid.

Darüber hinaus können als Komponente F1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- Methylaminobutan, Alkanolamine wie N-Aminoethylethanolamin, Ethanolamin, 3-Aminopropanol, Neopentanolamin.

Ferner können als Komponente F1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugte Verbindungen der Komponente F1) sind 1,2-Ethylendiamin, 1,4-Diaminobutan und Isophorondiamin.

Unter anionisch bzw. potentiell anionisch hydrophilierenden Verbindungen der Komponente F2) werden sämtliche Verbindungen verstanden, die mindestens eine isocyanatreaktive Gruppe, bevorzugt eine Amino-Gruppe aufweisen, sowie mindestens eine Funktionalität wie z.B. -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-ß-alanin, 2-(2-Aminoethylamino)ethansulfonsäure, Ethylendiaminpropyl- oder -butylsulfonsäure, 1,2- oder 1,3-Propylendiamin-ß-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann Cyclohexylaminopropansulfonsäure (CAPS) aus WO-A 01/88006 als anionisches oder potentiell anionisches Hydrophilierungsmittel verwendet werden.

Bevorzugte anionische oder potentiell anionische Hydrophilierungsmittel der Komponente F2) sind solche der vorstehend genannten Art, die über Carboxylat- bzw. Carobonsäuregruppen und/oder Sulfonatgruppen verfügen wie die Salze von N-(2-Aminoethyl)-ß-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen bzw. potentiell anionischen Hydrophilierungsmitteln und nichtionischen Hydrophilierungsmitteln verwendet werden.

In einer bevorzugten Ausführungsform zur Herstellung der speziellen Polyurethan-Dispersionen werden die Komponenten E1) bis E4) und F1) bis F2) in den folgenden Mengen eingesetzt, wobei sich die Einzelmengen stets zu 100 Gew.-% addieren:
5 bis 40 Gew.-% Komponente E1),
55 bis 90 Gew.-% E2),
0,5 bis 20 Gew.-% Summe der Komponenten E3) und F1)
0,1 bis 25 Gew.-% Summe der Komponenten E4) und F2), wobei bezogen auf die Gesamtmengen der Komponenten E1) bis E4) und F1) bis F2) 0,1 bis 5 Gew.-% an anionischen bzw. potentiell anionischen Hydrophilierungsmitteln aus E4) und/oder F2) verwendet werden.

In einer besonders bevorzugten Ausführungsform zur Herstellung der speziellen Polyurethan-Dispersionen werden die Komponenten E1) bis E4) und F1) bis F2) in den folgenden Mengen eingesetzt, wobei sich die Einzelmengen stets zu 100 Gew.-% aufaddieren:
5 bis 35 Gew.-% Komponente E1),
60 bis 90 Gew.-% E2),
0,5 bis 15 Gew.-% Summe der Komponenten E3) und F1)
0,1 bis 1Gew.-% Summe der Komponenten E4) und F2), wobei bezogen auf die Gesamtmengen der Komponenten E1) bis E4) und F1) bis F2) 0,2 bis 4 Gew.-% an anionischen bzw. potentiell anionischen Hydrophilierungsmitteln aus E4) und/oder F2) verwendet werden.

In einer ganz besonders bevorzugten Ausführungsform zur Herstellung der speziellen Polyurethan-Dispersionen werden die Komponenten E1) bis E4) und F1) bis F2) in den folgenden Mengen eingesetzt, wobei sich die Einzelmengen stets zu 100 Gew.-% aufaddieren:
10 bis 30 Gew.-% Komponente E1),
65 bis 85 Gew.-% E2),
0,5 bis 14 Gew.-% Summe der Komponenten E3) und F1)
0,1 bis 13,5 Gew.-% Summe der Komponenten E4) und F2), wobei bezogen auf die Gesamtmengen der Komponenten E1) bis E4) und F1) bis F2) 0,5 bis 3,0 Gew.-% an anionischen bzw. potentiell anionischen Hydrophilierungsmitteln aus E4) und/oder F2) verwendet werden.

Die Herstellung der anionisch hydrophilierten Polyurethan-Dispersionen (II) kann in einer oder mehreren Stufe/-n in homogener oder bei mehrstufiger Umsetzung, teilweise in disperser Phase durchgeführt werden. Nach vollständig oder teilweise durchgeführter Polyaddition aus E1) bis E4) erfolgt ein Dispergier-, Emulgier- oder Lösungsschritt. Im Anschluss erfolgt gegebenenfalls eine weitere Polyaddition oder Modifikation in disperser oder gelöster (homogener) Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach dem Aceton-Verfahren gearbeitet.

Für die Herstellung nach dem Aceton-Verfahren werden üblicherweise die Bestandteile E2) bis E4) und die Polyisocyanatkomponente E1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von, N-Methylpyrrolidon, N-Ethylpyrrolidon in der Dispersion verbleiben. Bevorzugt werden aber außer den üblichen aliphatischen, ketofunktionellen Lösemitteln keine anderen Lösungsmittel verwendet.

Anschließend werden die gegebenenfalls zu Beginn der Reaktion noch nicht zugegebenen Bestandteile von E1) bis E4) zudosiert.

Bei der Herstellung des Polyurethan-Prepolymeren aus E1) bis E4) beträgt das Stoffmengenverhältnis von Isocyanatgruppen zu mit isocyanatreaktiven Gruppen 1,05 bis 3,5, bevorzugt 1,2 bis 3,0, besonders bevorzugt 1,3 bis 2,5.

Die Umsetzung der Komponenten E1) bis E4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-Methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe F) werden NH₂- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine F1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition F2) mit NH₂- oder NH-Gruppen eingesetzt, erfolgt die Kettenverlängerung der Prepolymere bevorzugt vor der Dispergierung.

Die aminischen Komponenten F1) und F2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in F) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene organischen Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (II) beträgt typischerweise weniger als 1,0 Gew.-%, bevorzugt weniger als 0,5 Gew.-%, bezogen auf die gesamte Dispersion.

Der pH-Wert der erfindungswesentlichen Polyurethan-Dispersionen (II) beträgt typischerweise weniger als 9,0, bevorzugt weniger als 8,5, besonders bevorzugt weniger als 8,0 und liegt ganz besonders bevorzugt bei 6,0 bis 7,5.

Der Feststoffgehalt der Polyurethan-Dispersionen (II) beträgt typischerweise 40 bis 70, bevorzugt 50 bis 65, besonders bevorzugt 55 bis 65 Gew.-%.

Zur Verbesserung der Schaumbildung, Schaumstabilität oder der Eigenschaften des resultierenden Polymerschaums, können neben Additiv (I) noch weitere Additive in der Komponente (I) enthalten sein. Solche weiteren Additive können grundsätzlich alle an sich bekannten anionischen, nichtionischen, wie beispielsweise EO/PO-Blockcopolymere, und kationischen Tenside sein. Bevorzugt wird jedoch allein Komponente (I) eingesetzt.

Neben den Polymeren (II) und den erfindungswesentlichen Polyurethanen (I) können auch weitere Hilfs- und Zusatzstoffe (III) mitverwendet werden.

Beispiele für solche Hilfs- und Zusatzstoffe (III) sind Vernetzter, Verdicker bzw. Thixotropiermittel, andere wässrige Bindemittel, Antioxidantien, Lichtschutzmittel, Emulgatoren, Weichmacher, Pigmente, Füllstoffe und/oder Verlaufshilfsmittel.

Als Vernetzer können beispielsweise zugesetzt werden unblockierte Polyisocyanate, Amid- und Amin-Formaldehydharze, Phenolharze, Aldehyd- und Ketonharze, wie z.B. Phenol-Formaldehydharze, Resole, Furanharze, Harnstoffharze, Carbamidsäureesterharze, Triazinharze, Melaminharze, Benzoguanaminharze, Cyanamidharze oder Anilinharze enthalten sein.

Obgleich bereits die Tenside (I) die Polymere (II) merklich verdicken können, ist es möglich, weitere handelsübliche Verdicker einzusetzen, wie beispielsweise Dextrin-, Stärke- oder Cellulosederivate wie Celluloseether oder Hydroxyethylcellulose, organische vollsynthetische Verdicker auf Basis von Polyacrylsäuren, Polyvinylpyrrolidonen, Poly(meth)acrylverbindungen oder Polyurethanen (assoziative Verdicker) sowie anorganische Verdicker, wie Betonite oder Kieselsäuren.

Andere wässrige Bindemittel können z. B. aus Polyester-, Polyacrylat-, Polyepoxid- oder anderen Polyurethanpolymeren aufgebaut sein. Auch die Kombination mit strahlenhärtbaren Bindemitteln, wie sie z. B. in der EP-A-0 753 531 beschrieben sind, ist möglich. Ferner können auch andere anionische oder nicht-ionische Dispersionen, wie Polyvinylacetat-, Polyethylen-, Polystyrol-, Polybutadien-, Polyvinylchlorid-, Polyacrylat- und Copolymerisat-Dispersionen eingesetzt werden.

Die erfindungswesentlichen Zusammensetzungen enthalten, bezogen auf Trockensubstanz, typischerweise 80 bis 99,9 Gewichtsteile eines Polymers (II) und 0,1 bis 20 Gewichtsteile des Tensids (I). Bevorzugt enthalten die Zusammensetzungen, bezogen auf Trockensubstanz, 85 bis 99,5 Gewichtsteile eines Polymers (II) und 0,5 bis 15 Gewichtsteile des Tensids (I), besonders bevorzugt 90 bis 99 Gewichtsteile eines Polymers (II) und 1 bis 10 Gewichtsteile des Tensids (I) und ganz besonders bevorzugt 94 bis 99 Gewichtsteile eines Polymers (II und 1 bis 6 Gewichtsteile des Tensids (I).

Die weiteren als Hilfs- und Zusatzstoffe (III) zugegebenen Additive werden typischerweise in Mengen von 0 bis 10 Gewichtsteilen, bevorzugt 0 bis 5 Gewichtsteilen und besonders bevorzugt 0 bis 1,5 Gewichtsteilen in der erfindungsgemäßen Zusammensetzung eingesetzt.

Die Zugabe der Tenside (I) und der gegebenenfalls ebenso einzusetzenden weiteren Additive zum Polymer (II) kann in beliebiger Reihenfolge erfolgen. Die vorgenannten Additive können dabei gegebenenfalls in einem Lösungsmittel wie Wasser gelöst oder dispergiert eingesetzt werden.

Grundsätzlich ist es auch möglich, im Rahmen der Hilfs- und Zusatzstoffe durch Zugabe von Koagulantien eine Koagulation des Schaums herbeizuführen. Hierzu sind prinzipiell alle mehrfach kationisch funktionellen Verbindungen geeignet.

Das Aufschäumen im erfindungsgemäßen Verfahren kann durch Schütteln der Zusammensetzung, mechanisches Rühren oder durch Entspannung eines Treibgases erfolgen.

Das mechanische Aufschäumen kann mit beliebigen mechanischen Rühr-, Misch- und Dispergiertechniken unter Eintrag der zur Aufschäumung notwendigen Energie erfolgen. In der Regel wird hierbei Luft eingetragen, aber auch Stickstoff und andere Gase können hierfür benutzt werden.

Der so erhaltene Schaum wird beim Aufschäumen oder danach auf ein Substrat aufgetragen oder in eine Form gegeben und getrocknet.

Der Auftrag kann beispielsweise durch Gießen oder Rakeln erfolgen, aber auch andere an sich bekannte Techniken sind möglich. Ein mehrschichtiger Auftrag mit zwischengeschalteten Trocknungsschritten ist grundsätzlich auch möglich.

Eine befriedigende Trocknungsgeschwindigkeit der Schäume wird bereits bei 20°C beobachtet. Für eine schnellere Trocknung und Fixierung der Schäume werden jedoch bevorzugt Temperaturen oberhalb von 30°C benutzt. Bei der Trocknung sollten jedoch Temperaturen von 200°C nicht überschritten werden, da es anderenfalls u.a. zu unerwünschter Vergilbung der Schäume kommen kann. Bevorzugt werden Temperaturen von 60°C bis 180°C, besonders bevorzugt von 100°C bis 160°C benutzt. Möglich ist auch eine zwei- oder mehrstufige Trocknung. Die Trocknung erfolgt in der Regel unter Verwendung von an sich bekannten Heiz- und Trockenapparaten, z.B. (Umluft-) Trockenschränken. Der Auftrag sowie die Trocknung können jeweils diskontinuierlich oder kontinuierlich durchgeführt werden, bevorzugt ist jedoch ein gänzlich kontinuierliches Verfahren.

Die Trocknung erfolgt in der Regel unter Verwendung von an sich bekannten Heiz- und Trockenapparaten, wie (Umluft-) Trockenschränken, Heißluft oder IR-Strahlem. Auch die Trocknung durch Führen des beschichteten Substrates über geheizte Oberflächen, z.B. Walzen, ist möglich.

Der Auftrag sowie die Trocknung können jeweils diskontinuierlich oder kontinuierlich durchgeführt werden, bevorzugt ist jedoch ein gänzlich kontinuierliches Verfahren.

Als Substrate geeignet sind insbesondere Papiere oder Folien, die ein einfaches Ablösen der Schäume vor ihrem Einsatz z.B. als Wundauflage zur Abdeckung einer verletzten Stelle ermöglichen.

Die Schäume haben vor ihrer Trocknung typischerweise Schaumdichten von 50 bis 800 g/Liter, bevorzugt 100 bis 500 g/Liter, besonders bevorzugt 100 bis 250 g/Liter (Masse aller Einsatzstoffe [in g] bezogen auf das Schaumvolumen von einem Liter).

Die Schäume besitzen nach ihrer Trocknung eine mikroporöse, zumindest teilweise offenporige Struktur mit miteinander kommunizierenden Zellen. Die Dichte der getrockneten Schäume liegt dabei typischerweise unterhalb von 0,4 g/cm³, bevorzugt ist sie geringer als 0,35 g/cm³, besonders bevorzugt 0,01 bis 0,3 g/cm³ und ganz besonders bevorzugt 0,1 bis 0,3 g/cm³.

Das Absorptionsvermögen gegenüber physiologischer Salzlösung beträgt bei den Schäumen typischerweise 100 bis 1500 %, bevorzugt 300 bis 1500 %, besonders bevorzugt 300 bis 1000 % (Masse der aufgenommen Flüssigkeit bezogen auf die Masse des trockenen Schaums; Bestimmung nach DIN EN 13726-1, Teil 3.2). Die Durchlässigkeit gegenüber Wasserdampf beträgt typischerweise 1000 bis 8000 g/24 h * m², bevorzugt 1000 bis 6000 g/24 h * m², besonders bevorzugt 2000 bis 5000 g/24 h * m² (Bestimmung nach DIN EN 13726-2, Teil 3.2).

Die Schäume weisen eine gute mechanische Festigkeit und hohe Elastizität auf. Typischerweise sind die Werte für die Zugfestigkeit größer als 0,2 N/mm² und die Bruchdehnung ist größer als 250 %. Bevorzugt ist die Zugfestigkeit größer als 0,4 N/mm² und die Bruchdehnung größer als 350 % (Bestimmung nach DIN 53504).

Die Schäume haben nach dem Trocknen typischerweise eine Dicke von 0,1 mm bis 50 mm, bevorzugt 0,5 mm bis 20 mm, besonders bevorzugt 1 bis 10 mm, ganz besonders bevorzugt 1 bis 6 mm.

Die Schäume können überdies mit weiteren Materialien verklebt, laminiert oder beschichtet werden, beispielsweise auf Basis von Hydrogelen, (semi-) permeablen Folien, Beschichtungen, Hydrokolloiden oder anderen Schäumen.

Aufgrund ihrer vorteilhaften Eigenschaften werden die erfindungsgemäßen Polyurethan-Schäume bevorzugt als Wundauflagen oder für kosmetische Zwecke eingesetzt. Wundauflagen aus Polyurethan-Schäumen im Sinne der Erfindung sind poröse Materialien, bevorzugt mit zumindest teilweise vorhandener Offenzelligkeit, welche im Wesentlichen aus Polyurethanen bestehen und Wunden im Sinne einer sterilen Abdeckung vor Keimen bzw. Umgebungseinflüssen schützen, eine hohe Absorption von physiologischer Salzlösung bzw. Wundflüssigkeit aufweisen, durch geeignete Feuchtdurchlässigkeit für ein geeignetes Wundklima sorgen und eine ausreichende mechanische Festigkeit aufweisen.

Sofern es zweckdienlich ist, kann im erfindungsgemäßen Verfahren ein Schritt zur Sterilisation erfolgen. Ebenso ist es grundsätzlich möglich, nach dem erfindungsgemäßen Verfahren erhältlichen Wundauflagen nach deren Herstellung zu sterilisieren. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung beispielsweise durch Gammabestrahlung erfolgt.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

Bevorzugte Wirkstoffe der vorgenannten Art sind solche aus der Gruppe der Antiseptika, Wachstumsfaktoren, Proteaseinhibitoren und nichtsteroidalen Antiphlogistika/Opiate oder auch Wirkstoffe wie beispielsweise Thrombin alfa zur lokalen Blutstillung.

In einer bevorzugten Ausführungsform der vorliegenden Erfindung umfasst der Wirkstoff ein antiseptisches Biguanid und/oder dessen Salz, vorzugsweise das Hydrochlorid.

Als Biguanide werden Verbindungen bezeichnet, die sich vom Biguanid (C₂H₇N₅) ableiten, insbesondere dessen Polymere. Antiseptische Biguanide sind solche Verbindungen, die eine antimikrobielle Wirkung aufweisen, also als Bakteriostatika oder vorzugsweise als Bakterizide wirken. Die Verbindungen weisen vorzugsweise eine breite Wirkung gegen viele Bakterien auf und können durch eine Wirkung gegen E. coli von mindestens 0,5 µg/ml, vorzugsweise mindestens 12 oder mindestens 25 µg/ml gekennzeichnet werden (minimal mikrobizide Konzentration oder MMK, gemessen im Suspensionsversuch).

Ein bevorzugtes antiseptisches Biguanid gemäß dieser Erfindung ist Poly(imino[iminocarbonyl]iminopolymethylen), wobei die Verwendung von Poly(hexamethylen)biguanid (PHMB), das auch als Polyhexanid bezeichnet wird, als antiseptisches Biguanid besonders bevorzugt ist.

Der Begriff "antiseptische Biguanide" gemäß dieser Erfindung beinhaltet auch Metaboliten und/oder Prodrugs der antiseptischen Biguaniden. Antiseptische Biguanide können dabei als Racemate oder reine Isoformen vorliegen.

Bevorzugt enthalten die geschäumten Artikel aus Polyurethan-Schäumen beziehungsweise die Zusammensetzungen gemäß der vorliegenden Erfindung antiseptisches Biguanid und/oder dessen Salz, vorzugsweise das Hydrochlorid, in einer Konzentration von 0,01 bis 20 Gew-%, wobei die Konzentration von 0,1 bis 5 Gew-% besonders vorteilhaft ist. Das Biguanid kann eine beliebige Molekulargewichtsverteilung aufweisen.

Ein weiterer Gegenstand der vorliegenden Erfindung sind die nach dem erfindungsgemäßen Verfahren erhältlichen Polymerschäume, insbesondere Polyurethanschäume sowie deren Verwendung als Wundauflage sowie im kosmetischen Bereich. Bevorzugt ist die Verwendung als Wundauflage.

### Beispiele:

Sofern nicht abweichend gekennzeichnet, beziehen sich alle Prozentangaben auf das Gewicht. Die für die Schaumadditive angegebenen Gehalte beziehen sich auf wässrige Lösungen.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

Die Bestimmung der mittleren Teilchengröße (angegeben ist das Zahlenmittel) der Polyurethan-Dispersion 1 erfolgte mittels Laserkorrelations-Spektroskopie (Gerät: Malvern Zetasizer 1000, Malvern Inst. Limited).

Die eingesetzten Polypropylenglykol-Polyethers wurden, falls nicht anders erwähnt, mittels DMC-Katalyse (basenfrei) hergestellt.

Bei den angegebenen Molmassen handelt es sich, falls nicht anders erwähnt, um gewichtsmittlere Molmassen. Sie wurden durch GPC-Analyse in Tetrahydrofuran bei einer Flussrate von 0,6 ml/min bestimmt. Die Kalibrierung erfolgte durch Polystyrol-Standards.

### Verwendete Substanzen und Abkürzungen:

| | |
|---|---|
| Diaminosulfonat: | NH₂-CH₂CH₂-NH-CH₂CH₂-SO₃Na (45 %ig in Wasser) |
| Desmophen^{®} C2200: | Polycarbonatpolyol, OH-Zahl 56 mg KOH/g, zahlenmittleres Molekulargewicht 2000 g/mol (Bayer MaterialScience AG, Leverkusen, DE) |
| PolyTHF^{®} 2000: | Polytetramethylenglykolpolyol, OH-Zahl 56 mg KOH/g, zahlenmittleres Molekulargewicht 2000 g/mol (BASF AG, Ludwigshafen, DE) |
| PolyTHF^{®} 1000: | Polytetramethylenglykolpolyol, OH-Zahl 112 mg KOH/g, zahlenmittleres zahlenmittleres Molekulargewicht 1000 g/mol (BASF AG, Ludwigshafen, DE) |
| Polyether LB 25: | monofunktioneller Polyether auf Ethylenoxid-/Propylenoxidbasis, zahlenmittleres Molekulargewicht 2250 g/mol, OH-Zahl 25 mg KOH/g (Bayer MaterialScience AG, Leverkusen, DE) |
| HDI: | Hexamethylen-1,6-diisocyanat |

### Beispiel 1: Herstellung der Polyurethan-Dispersion 1

1077,2 g PolyTHF^{®} 2000, 409,7 g PolyTHF^{®} 1000, 830,9 g Desmophen^{®} C2200 und 48,3 g Polyether LB 25 wurden in einer Standard-Rührapparatur auf 70 °C aufgeheizt. Anschließend wurde bei 70 °C innerhalb von 5 min ein Gemisch aus 258,7 g Hexamethylendiisocyanat und 341,9 g Isophorondiisocyanat zugegeben und bei 120°C gerührt, bis der theoretische NCO-Wert erreicht oder leicht unterschritten war. Das fertige Prepolymer wurde mit 4840 g Aceton gelöst und dabei auf 50 °C abgekühlt und anschließend wurde eine Lösung aus 27,4 g Ethylendiamin, 127,1 g Isophorondiamin, 67,3 g Diaminosulfonat und 1200 g Wasser innerhalb von 10 min zudosiert. Die Nachrührzeit betrug 10 min. Dann wurde durch Zugabe von 654 g Wasser dispergiert. Es folgte die Entfernung des Lösemittels durch Destillation im Vakuum.

Die erhaltene Polyurethan-Dispersion hatte nachfolgende Eigenschaften:

| | |
|---|---|
| Feststoffgehalt: | 59,0% |
| Partikelgröße (LKS): | 487 nm |
| pH (23°C): | 7,1 |

### Beispiel 2 (Erfindungsgemäß):

### Herstellung eines Tensids (I) auf Basis eines difunktionellen Isocyanat-Prepolymers

1300 g HDI, 1,3 g Benzoylchlorid und 1,3 g para-Toluolsulfonsäuremethylester wurden in einem 4 Liter Vierhalskolben unter Rühren vorgelegt. Innerhalb von 3 Stunden fügte man bei 80°C 1456 g eines difunktionellen Polypropylenglykol-Polyethers mit einem zahlenmittleren Molekulargewicht von 2000 g/mol hinzu und rührte 1 Stunde bei gleicher Temperatur nach. Anschließend wurde durch Dünnschichtdestillation bei 130°C und 0,1 Torr das überschüssige HDI abdestilliert, im Vorlagekolben befand sich 1 g Chlorpropionsäure. Das erhaltene NCO-Prepolymer hatte einen NCO-Gehalt von 3,23% und einer Viskosität von 1650 mPas (25°C).

In einem 21 Vierhalskolben wurden unter Rühren 225 g Polyether LB 25 und 100 g eines difunktionellen Polyethylenglykol-Polyethers mit einem zahlenmittleren Molekulargewicht von 2000 g/mol vorgelegt. Innerhalb von 2,5 Stunden fügte man bei 80°C 260 g des oben genannten NCO-Prepolymers hinzu und rührte 4 Stunden bei gleicher Temperatur nach, bis IR-spektroskopisch keine NCO-Gruppen mehr nachweisbar sind. Das erhaltene Tensid war ein Feststoff mit einer gewichtsmittleren Molmasse von 21346 g/mol.

### Beispiel 3 (Erfindungsgemäß):

### Herstellung eines Tensids (I) auf Basis eines difunktionellen Isocyanat-Prepolymers

2000 g HDI, 1,3 g Benzoylchlorid und 1,3 g *para*-Toluolsulfonsäuremethylester wurden in einem 41 Vierhalskolben unter Rühren vorgelegt. Innerhalb von 3 Stunden fügte man bei 80°C 1000 g eines difunktionellen Polypropylenglykol-Polyethers mit einem zahlenmittleren Molekulargewicht von 1000 g/mol hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 6,24% und einer Viskosität von 1650 mPas (25°C).

In einem 21 Vierhalskolben wurden unter Rühren 600 g eines monofunktionellen Polyethylenglykol-Polyethers (MeOPEG) mit einem zahlenmittleren Molekulargewicht von 2000 g/mol vorgelegt. Innerhalb von 0,5 Stunden fügte man bei 70°C 202 g des oben genannten NCO-Prepolymers hinzu und rührte 4 Stunden bei 80°C nach, bis IR-spektroskopisch keine NCO-Gruppen mehr nachweisbar waren. Das erhaltene Tensid war ein Feststoff mit einer gewichtsmittleren Molmasse von 7232 g/mol.

### Beispiel 4 (Erfindungsgemäß):

### Herstellung eines Tensids (I) auf Basis eines difunktionellen Isocyanat-Prepolymers

2000 g HDI, 1,3 g Benzoylchlorid und 1,3 g *para*-Toluolsulfonsäuremethylester wurden in einem 41 Vierhalskolben unter Rühren vorgelegt. Innerhalb von 3 Stunden fügte man bei 80°C 1000 g eines difunktionellen Polypropylenglykol-Polyethers mit einem zahlenmittleren Molekulargewicht von 1000 g/mol hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 6,24% und einer Viskosität von 1650 mPas (25°C).

In einem 2 1 Vierhalskolben wurden unter Rühren 750 g eines monofunktionellen Polyethylenglykol-Polyethers (MeOPEG) mit einem zahlenmittleren Molekulargewicht von 5000 g/mol vorgelegt. Innerhalb von 0,5 Stunden fügte man bei 70°C 101 g des oben genannten NCO-Prepolymers hinzu und rührte 4 Stunden bei 80°C nach, bis IR-spektroskopisch keine NCO-Gruppen mehr nachweisbar waren. Das erhaltene Tensid war ein Feststoff mit einer gewichtsmittleren Molmasse von 13849 g/mol.

### Beispiel 5 (Erfindungsgemäß):

### Herstellung eines Tensids (I) auf Basis eines difunktionellen Isocyanat-Prepolymers

2000 g HDI, 1,3 g Benzoylchlorid und 1,3 g *para*-Toluolsulfonsäuremethylester wurden in einem 41 Vierhalskolben unter Rühren vorgelegt. Innerhalb von 3 Stunden fügte man bei 80°C 1000 g eines difunktionellen Polypropylenglykol-Polyethers mit einem zahlenmittleren Molekulargewicht von 1000 g/mol hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 6,24% und einer Viskosität von 1650 mPas (25°C).

In einem 21 Vierhalskolben wurden unter Rühren 675 g Polyether LB 25 vorgelegt. Innerhalb von 0,5 Stunden fügte man bei 70°C 202 g des oben genannten NCO-Prepolymers hinzu und rührte 5 Stunden bei 90°C nach, bis IR-spektroskopisch keine NCO-Gruppen mehr nachweisbar waren. Das erhaltene Tensid hatte eine Viskosität von 5750 mPas (25°C) und eine gewichtsmittlere Molmasse von 9511 g/mol.

### Beispiel 6 (Erfindungsgemäß):

### Herstellung eines Tensids (I) auf Basis eines difunktionellen Isocyanat-Prepolymers

2000 g HDI, 1,3 g Benzoylchlorid und 1,3 g *para*-Toluolsulfonsäuremethylester wurden in einem 41 Vierhalskolben unter Rühren vorgelegt. Innerhalb von 3 Stunden fügte man bei 80°C 1000 g eines difunktionellen Polypropylenglykol-Polyethers mit einem zahlenmittleren Molekulargewicht von 1000 g/mol hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 6,24% und einer Viskosität von 1650 mPas (25°C).

In einem 21 Vierhalskolben wurden unter Rühren 281 g Polyether LB 25 und 125 g eines difunktionellen Polyethylenglykol-Polyethers mit einem zahlenmittleren Molekulargewicht von 2000 g/mol vorgelegt. Innerhalb von 2,5 Stunden fügte man bei 80°C 167,5 g des oben genannten NCO-Prepolymers hinzu und rührte 5 Stunden 80 bis 100°C nach, bis IR-spektroskopisch keine NCO-Gruppen mehr nachweisbar waren. Das erhaltene Tensid war ein Feststoff.

### Beispiel 7 (Erfindungsgemäß):

### Herstellung eines Tensids (I) auf Basis eines difunktionellen Isocyanat-Prepolymers

In einem 21 Vierhalskolben wurden unter Rühren 337 g Polyether LB 25 und 150 g eines difunktionellen Polyethylenglykol-Polyethers mit einem zahlenmittleren Molekulargewicht von 2000 g/mol vorgelegt. Innerhalb von 2,5 Stunden fügte man bei 80°C 98,5 g Desmodur E 305 (Desmodur E 305 ist ein weitgehend lineares NCO-Prepolymer auf Basis Hexamethylendiisocyanat, NCO-Gehalt ca. 12,8%) hinzu und rührte 5 Stunden 90 bis 110°C nach, bis IR-spektroskopisch keine NCO-Gruppen mehr nachweisbar waren. Das erhaltene Tensid war ein Feststoff.

### Beispiel 8 (Erfindungsgemäß):

### Herstellung eines Tensids (I) auf Basis eines difunktionellen Isocyanat-Prepolymers

1300 g HDI, 1,3 g Benzoylchlorid und 1,3 g *para*-Toluolsulfonsäuremethylester wurden in einem 41 Vierhalskolben unter Rühren vorgelegt. Innerhalb von 3 Stunden fügte man bei 80°C 1456 g eines difunktionellen Polypropylenglykol-Polyethers mit einem zahlenmittleren Molekulargewicht von 2000 g/mol hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 3,23% und einer Viskosität von 1650 mPas (25°C).

In einem 21 Vierhalskolben wurden unter Rühren 100 g eines difunktionellen Polyethylenglykol-Polyethers mit einem zahlenmittleren Molekulargewicht von 2000 g/mol vorgelegt. Innerhalb von 2,5 Stunden fügte man bei 80°C 258 g des oben genannten NCO-Prepolymers hinzu und rührte 3 Stunden bei 100°C nach. Dann wurden 225 g Polyether LB 25 zugesetzt und 2,5 Stunden bei 115°C gerührt, bis IR-spektroskopisch keine NCO-Gruppen mehr nachweisbar waren. Das erhaltene Tensid war ein sehr viskose Flüssigkeit.

### Beispiel 9 (Erfindungsgemäß):

### Herstellung eines Tensids (I) auf Basis eines difunktionellen Isocyanat-Prepolymers

1300 g HDI, 1,3 g Benzoylchlorid und 1,3 g *para*-Toluolsulfonsäuremethylester wurden in einem 41 Vierhalskolben unter Rühren vorgelegt. Innerhalb von 3 Stunden fügte man bei 80°C 1456 g eines difunktionellen Polypropylenglykol-Polyethers mit einem zahlenmittleren Molekulargewicht von 2000 g/mol hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 3,23% und einer Viskosität von 1650 mPas (25°C).

In einem 21 Vierhalskolben wurden unter Rühren 112,5 g Polyether LB 25 und 150 g eines difunktionellen Polyethylenglykol-Polyethers mit einem zahlenmittleren Molekulargewicht von 2000 g/mol vorgelegt. Innerhalb von 0,5 Stunden fügte man bei 80°C 257 g des oben genannten NCO-Prepolymers hinzu und rührte 4 Stunden bei 100-115°C nach, bis IR-spektroskopisch keine NCO-Gruppen mehr nachweisbar waren. Das erhaltene Tensid war ein Feststoff.

### Beispiel 10 (Erfindungsgemäß):

### Herstellung eines Tensids (I) auf Basis eines difunktionellen Isocyanat-Prepolymers

1300 g HDI, 1,3 g Benzoylchlorid und 1,3 g *para*-Toluolsulfonsäuremethylester wurden in einem 4 l Vierhalskolben unter Rühren vorgelegt. Innerhalb von 3 Stunden fügte man bei 80°C 1456 g eines difunktionellen Polypropylenglykol-Polyethers mit einem zahlenmittleren Molekulargewicht von 2000 g/mol hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 3,23% und einer Viskosität von 1650 mPas (25°C).

In einem 21 Vierhalskolben wurden unter Rühren 287 g Polyether LB 25 und 42,5 g eines difunktionellen Polyethylenglykol-Polyethers mit einem zahlenmittleren Molekulargewicht von 2000 g/mol vorgelegt. Innerhalb von 0,5 Stunden fügte man bei 80°C 220 g des oben genannten NCO-Prepolymers hinzu und rührte 5 Stunden bei 100-120°C nach, bis IR-spektroskopisch keine NCO-Gruppen mehr nachweisbar waren. Das erhaltene Tensid war eine sehr hochviskose Flüssigkeit.

### Beispiel 11 Erfindungsgemäß):

### Herstellung eines Tensids (I) auf Basis eines difunktionellen Isocyanat-Prepolymers

1300 g HDI, 1,3 g Benzoylchlorid und 1,3 g *para*-Toluolsulfonsäuremethylester wurden in einem 4 l Vierhalskolben unter Rühren vorgelegt. Innerhalb von 3 Stunden fügte man bei 80°C 1456 g eines difunktionellen Polypropylenglykol-Polyethers mit einem zahlenmittleren Molekulargewicht von 2000 g/mol hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 3,23% und einer Viskosität von 1650 mPas (25°C).

In einem 2 l Vierhalskolben wurden unter Rühren 200 g eines monofunktionellen Polyethylenglykol-Polyethers (MeOPEG) mit einem zahlenmittleren Molekulargewicht von 2000 g/mol und 100 g eines difunktionellen Polyethylenglykol-Polyethers mit einem zahlenmittleren Molekulargewicht von 2000 g/mol vorgelegt. Innerhalb von 0,5 Stunden fügte man bei 80°C 257 g des oben genannten NCO-Prepolymers hinzu und rührte 4 Stunden bei 100-120°C nach, bis IR-spektroskopisch keine NCO-Gruppen mehr nachweisbar waren. Das erhaltene Tensid war ein Feststoff.

### Beispiel 12 (Erfindungsgemäß):

### Herstellung eines Tensids (I) auf Basis eines difunktionellen Isocyanat-Prepolymers

1300 g HDI und 0,3 g Dibutylphosphat wurden in einem 4 1 Vierhalskolben unter Rühren vorgelegt. Innerhalb von 3 Stunden fügte man bei 80°C 1456 g eines difunktionellen Polypropylenglykol-Polyethers mit einem zahlenmittleren Molekulargewicht von 2000 g/mol hinzu und rührte 1 Stunde bei gleicher Temperatur nach. Anschließend wurde durch Dünnschichtdestillation bei 130°C und 0,1 Torr das überschüssige HDI abdestilliert, im Vorlagekolben befinden sich 2 g Ronotec 201 (Tocopherol). Das erhaltene NCO-Prepolymer hatte einen NCO-Gehalt von 3,27% und einer Viskosität von 1680 mPas (25°C).

In einem 21 Vierhalskolben wurden unter Rühren 225 g Polyether LB 25 und 100 g eines difunktionellen Polyethylenglykol-Polyethers mit einem zahlenmittleren Molekulargewicht von 2000 g/mol vorgelegt. Innerhalb von 2,5 Stunden fügte man bei 70°C 260 g des oben genannten NCO-Prepolymers hinzu und rührte 5 Stunden bei gleicher Temperatur nach, bis IR-spektroskopisch keine NCO-Gruppen mehr nachweisbar waren. Das erhaltene Tensid war ein Feststoff.

### Beispiel 13 (Erfindungsgemäß):

### Herstellung eines Tensids (I) auf Basis eines difunktionellen Isocyanat-Prepolymers

1300 g HDI wurden in einem 4 1 Vierhalskolben unter Rühren vorgelegt. Innerhalb von 3 Stunden fügte man bei 80°C 1456 g eines difunktionellen Polypropylenglykol-polyethylenglykol-Polyethers mit einem zahlenmittleren Molekulargewicht von 2000 g/mol und einem Anteil an EthylenoxidEinheiten von 24 Gew.-% hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 1,99% und einer Viskosität von 1040 mPas (25°C).

In einem 21 Vierhalskolben wurden unter Rühren 169 g Polyether LB 25 und 75,0 g eines difunktionellen Polyethylenglykol-Polyethers mit einem zahlenmittleren Molekulargewicht von 2000 g/mol vorgelegt. Innerhalb von 2,5 Stunden fügte man bei 70°C 284 g des oben genannten NCO-Prepolymers hinzu und rührte 5 Stunden bei bis zu 110°C nach, bis IR-spektroskopisch keine NCO-Gruppen mehr nachweisbar waren. Das erhaltene Tensid war ein Feststoff.

### Beispiel 14 (Erfindungsgemäß):

### Herstellung eines Tensids (I) auf Basis eines difunktionellen Isocyanat-Prepolymers

1400 g HDI und 0,2 g Isophthaloylchlorid wurden in einem 4 l Vierhalskolben unter Rühren vorgelegt. Innerhalb von 3 Stunden fügte man bei 80°C 1400 g eines difunktionellen Polypropylenglykol-Polyethers mit einem zahlenmittleren Molekulargewicht von 2000 g/mol (hergestellt durch KOH-katalysierte Polymerisation) hinzu und rührte 1 Stunde bei gleicher Temperatur nach. Anschließend wurde durch Dünnschichtdestillation bei 130°C und 0,1 Torr das überschüssige HDI abdestilliert. Das erhaltene NCO-Prepolymer hatte einen NCO-Gehalt von 3,6% und einer Viskosität von 1480 mPas (25°C).

In einem 21 Vierhalskolben wurden unter Rühren 225 g Polyether LB 25 und 100 g eines difunktionellen Polyethylenglykol-Polyethers mit einem zahlenmittleren Molekulargewicht von 2000 g/mol vorgelegt. Innerhalb von 2,5 Stunden fügte man bei 70°C 233 g des oben genannten NCO-Prepolymers hinzu und rührte 3 Stunden bei max 115°C nach, bis IR-spektroskopisch keine NCO-Gruppen mehr nachweisbar waren. Das erhaltene Tensid war ein Feststoff.

### Beispiele S1-S5 (Erfindungsgemäß):

### Herstellung von Schäumen aus der Polyurethan-Dispersion 1 und eines Tensids (I)

Wie in Tabelle 1 angegeben, wurden je 120 g der Polyurethan-Dispersion 1, hergestellt nach Beispiel 1, mit verschiedenen (Schaum-) Additiven vermischt und unter Verwendung eines handelsüblichen Handrührgerätes (Rührer aus gebogenem Draht) auf 0,5 Liter Schaumvolumen aufgeschlagen. Danach wurden die Schäume mittels eines Filmziehgerätes (Rakel) mit Spalthöhe 6 mm auf nicht-haftendes Papier aufgezogen und für 20 Minuten bei 120°C getrocknet.

Es wurden durchweg reinweiße Schäume mit guten mechanischen Eigenschaften erhalten. Wie Tabelle 1 zu entnehmen ist, wurden durch Verwendung der speziellen (Schaum-) Additive 2, 5, 6, 10 und 12 Schäume mit hoher Aufnahmegeschwindigkeit von physiologischer Salzlösung und guter Saugleistung bei gleichzeitig feiner, homogener Porenstruktur erhalten.

Exemplarisch wurde gezeigt, dass der Schaum S5 gemäß Richtlinie ISO 10993.5 als nicht zytotoxisch einzustufen ist.

**Tabelle 1**

| Schaum | Beispiel # | Menge [g] (Gehalt [%]) | Aufnahmegeschwindigkeit¹⁾ [s] | Saugleistung²⁾ [g/100cm²] | Porosität/Schaumstruktur |
|---|---|---|---|---|---|
| S1 | 2 | 25,3 (15) | 48 | nicht ermittelt | sehr fein |
| S2 | 5 | 12,6 (30) | 4 | 30 | sehr fein |
| S3 | 6 | 12,4 (30) | 15 | 56 | mittel |
| S4 | 10 | 13,2 (28) | 28 | 46 | fein |
| S5 | 12 | 11,8 (25) | 44 | 47 | sehr fein |

| | | | | | |
|---|---|---|---|---|---|
| ¹⁾ Zeit bis zum vollständigen Eindringen von einem Milliliter der Prüflösung A, hergestellt wie in DIN EN 13726-1, Teil 3.2 beschrieben; Prüfung der Papier zugewandten Seite; ²⁾ Die Bestimmung der "freien Saugleistung" erfolgte nach DIN EN 13726-1, Teil 3.2. | | | | | |

### Vergleichsbeispiele V1-V2:

### Herstellung von Schäumen aus Polyurethan-Dispersion 1 und eines Tensids

Wie in Tabelle 2 angegeben, wurden jeweils 120 g der Polyurethan-Dispersion 1, hergestellt nach Beispiel 1, mit verschiedenen (Schaum-) Additiven vermischt und unter Verwendung eines handelsüblichen Handrührgerätes (Rührer aus gebogenem Draht) auf 0,5 Liter Schaumvolumen aufgeschlagen. Danach wurden die Schäume mittels eines Filmziehgerätes (Rakel) mit Spalthöhe 6 mm auf nicht-haftendes Papier aufgezogen und für 20 Minuten bei 120°C getrocknet.

Wie Tabelle 2 zu entnehmen ist, besitzen gemäß Prüfung nach Richtlinie ISO 10993.5 die Schäume V1 und V2 eine additiwerursachte, stark zytotoxische Wirkung: die Zellviabilitäten lagen bei diesen Schäumen unter 3%.

**Tabelle 2**

| Schaum | (Schaum-) Additive | | | | Porosität/Schaumstruktur | Zytotoxizität²⁾ |
|---|---|---|---|---|---|---|
| | Typ¹⁾ | Menge [g] | Typ¹⁾ | Menge [g] | | |
| V1 | A | 4,34 | B | 5,76 | sehr fein | stark zytotoxisch |
| V2 | A | 0,24 | C | 1,47 | fein, aber Narbenbildung & Volumenschrumpf | stark zytotoxisch |

| | | | | | | |
|---|---|---|---|---|---|---|
| ¹⁾ A: Ammoniumstearat (ca. 30 %, Stokal^{®} STA, Bozzetto GmbH, Krefeld, DE); B: Sulfosuccinamat (ca. 34 %, Stokal^{®} SR, Bozzetto GmbH, Krefeld, DE); C: C12-C16-Fettalkohol-Polyglykosid (ca. 51%, PlantaCare® 1200 UP, Cognis Deutschland GmbH & Co. KG, Düsseldorf, DE); ²⁾ Überprüfung gemäß Richtlinie ISO 10993.5 | | | | | | |

## Patentansprüche

1. Zusammensetzungen erhältlich durch Mischen von wenigstens Polyurethanen (I) mit einem Gehalt an freien Isocyanatgruppen von max. 1,0 Gew.-% und einem Gehalt an über monofunktionelle Alkohole B) eingebauten, innerhalb von Polyetherketten angeordneten Ethylenoxideinheiten (Molekulargewicht = 44 g/mol) von 10 bis 95 Gew.-%, die durch Umsetzung von
A) Polyisocyanat-Prepolymeren mit einer (mittleren) NCO-Funktionalität von größer oder gleich 1 mit
B) 10 bis 100 Äquivalent-%, bezogen auf die Isocyanatgruppen von A), einer einwertigen Alkoholkomponente, umfassend mindestens einen einwertigen Polyetheralkohol mit einem zahlenmittleren Molekulargewicht von 150 bis 5000 g/mol mit einem Gehalt an Oxyethyleneinheiten von 30 bis 100 Mol.-% bezogen auf den Gesamtgehalt an Oxyalkyleneinheiten des einwertigen Polyetheralkohols,
C) 0 bis 20 Äquivalent-%, bezogen auf die Isocyanatgruppen von A), einer einwertigen Alkoholkomponente, umfassend von der Verbindungen der Komponente B) verschiedene einwertige Alkohole mit einem zahlenmittleren Molekulargewicht von 32 bis 5000 g/mol,
D) 0 bis 80 Äquivalent-%, bezogen auf die Isocyanatgruppen von A), an im Sinne der NCO-Additionsreaktion mindestens difunktionellen Aufbaukomponenten mit einem zahlenmittleren Molekulargewicht von 32 bis 10000 g/mol
unter Urethan- und gegebenenfalls Harnstoffbildung hergestellt worden sind, wobei gegebenenfalls im Überschuss vorliegende NCO-Gruppen durch gleichzeitig oder anschließend erfolgende Sekundärreaktionen bis auf einen Restgehalt von maximal 1,0 Gew.-% abreagiert worden sind
und schäumbaren Polymeren (II),
**dadurch gekennzeichnet, dass** als schäumbare Polymere (II) anionisch hydrophilierte Polyurethane in Form wässriger Dispersionen eingesetzt werden.

2. Zusammensetzungen gemäß Anspruch 1, **dadurch gekennzeichnet, dass** in den Polyurethanen (Γ) der Gehalt an Ethylenoxideinheiten (Molekulargewicht = 44 g/mol) 45 bis 55 Gew.% bezogen auf das Polyurethan beträgt.

3. Zusammensetzungen gemäß Anspruch 1 oder 2, **dadurch gekennzeichnet, dass** die mittlere NCO-Funktionalität der Polyisocyanat-Prepolymere der Komponente A) 1,8 bis 2,2 beträgt.

4. Zusammensetzungen gemäß einem der Ansprüche 1 bis 3, **dadurch gekennzeichnet, dass** zur Herstellung der Verbindungen der Komponente A) Polyole mit OH-Funktionalitäten von 1,8 bis 2,5 eingesetzt werden.

5. Zusammensetzungen gemäß Anspruch 4, **dadurch gekennzeichnet, dass** als Polyole beim Aufbau der Verbindungen der Komponente A) Polyetherpolyole mit zahlenmittleren Molekulargewichten Mₐ von 300 bis 8000 g/mol eingesetzt werden.

6. Zusammensetzungen gemäß Anspruch 5, **dadurch gekennzeichnet, dass** die Polyetherpolyole eine Polydispersität (PD = M_{w}/Mₙ) von 1,0 bis 1,5 und eine OH-Funktionalität von größer 1,9 sowie einen Gehalt an ungesättigten Endgruppen von kleiner oder gleich 0,02 Milliäquivalenten pro Gramm Polyol (meq/g) (Bestimmungsmethode ASTM D2849-69) aufweisen.

7. Zusammensetzungen gemäß Anspruch 5, **dadurch gekennzeichnet, dass** bei der Herstellung der Polyurethane (I) in Komponente B) monohydroxyfunktionelle Polyalkylenoxidpolyetheralkohole eingesetzt werden, die im statistischen Mittel 5 bis 70 Ethylenoxideinheiten pro Molekül und 30 bis 100 mol-% Ethylenoxid- und 0 bis 70 mol-% Propylenoxideinheiten bezogen auf die Gesamtmenge an Oxyalkyleneinheiten aufweisen.

8. Zusammensetzungen gemäß Anspruch 1, **dadurch gekennzeichnet, dass** die anionisch hydrophilierten Polyurethane in Form wässriger Dispersionen erhältlich sind, indem
E) isocyanatfunktionelle Prepolymere mindestens aus
E1) organischen Polyisocyanaten
E2) polymeren Polyolen mit zahlenmittleren Molekulargewichten von 400 bis 8000 g/mol und OH-Funktionalitäten von 1,5 bis 6 und
E3) gegebenenfalls hydroxyfunktionellen Verbindungen mit Molekulargewichten von 62 bis 399 g/mol und
E4) gegebenenfalls isocyanatreaktiven, anionischen oder potentiell anionischen und/oder gegebenenfalls nichtionischen Hydrophilierungsmitteln,
hergestellt werden,
F) deren freie NCO-Gruppen dann ganz oder teilweise
F1) gegebenenfalls mit aminofunktionellen Verbindungen mit Molekulargewichten von 32 bis 400 g/mol und/oder
F2) isocyanatreaktiven, bevorzugt aminofunktionellen, anionischen oder potentiell anionischen Hydrophilierungsmitteln
unter Kettenverlängerung umgesetzt werden und die Prepolymere vor, während oder nach Schritt F) in Wasser dispergiert werden, wobei gegebenenfalls enthaltene potentiell ionische Gruppen durch teilweise oder vollständige Umsetzung mit einem Neutralisationsmittel in die ionische Form überführt werden.

9. Verfahren zur Herstellung von Polymerschäumen, bei dem eine Zusammensetzung gemäß einem der Ansprüche 1 bis 8 aufgeschäumt und getrocknet wird.

10. Schäume erhältlich nach einem Verfahren gemäß Anspruch 9.

11. Wundauflagen erhältlich unter Verwendung von Zusammensetzungen gemäß einem der Ansprüche 1 bis 8.

## Claims

1. Compositions obtainable by mixing at least polyurethanes (I) having a free isocyanate group content of not more than 1.0% by weight and a 10% to 95% by weight content of ethylene oxide units (molecular weight = 44 g/mol) incorporated via monofunctional alcohols B) and arranged within polyether chains, which have been prepared by reaction of
A) polyisocyanate prepolymers having an (average) NCO functionality of not less than 1 with
B) 10 to 100 equivalent%, based on the isocyanate groups of A), of a monohydric alcohol component comprising at least one monohydric polyether alcohol having a number average molecular weight in the range from 150 to 5000 g/mol and an oxyethylene units content of 30 to 100 mol%, based on the total content of oxyalkylene units in the monohydric polyether alcohol,
C) 0 to 20 equivalent%, based on the isocyanate groups of A), of a monohydric alcohol component comprising monohydric alcohols having a number average molecular weight in the range from 32 to 5000 g/mol which are other than the compounds of component B),
D) 0 to 80 equivalent%, based on the isocyanate groups of A), of constructional components having a number average molecular weight in the range from 32 to 10 000 g/mol which are at least difunctional for the purposes of the NCO addition reaction
with urethane formation and with or without urea formation, wherein any excess NCO groups have been reacted away, by simultaneous or subsequent secondary reactions, down to a residual content of not more than 1.0% by weight,
and foamable polymers (II),
**characterized in that** anionically hydrophilicized polyurethanes in the form of aqueous dispersions are used as foamable polymers (II).

2. Compositions according to Claim 1, **characterized in that** the polyurethanes (I) have a 45% to 55% by weight content, based on the polyurethane, of ethylene oxide units (molecular weight = 44 g/mol).

3. Compositions according to Claim 1 or 2, **characterized in that** the average NCO functionality of the polyisocyanate prepolymers of component A) is in the range from 1.8 to 2.2.

4. Compositions according to any one of Claims 1 to 3, **characterized in that** the compounds of component A) are prepared using polyols having OH functionalities in the range from 1.8 to 2.5.

5. Compositions according to Claim 4, **characterized in that** the polyols used to construct the compounds of component A) are polyether polyols having number average molecular weights Mₙ in the range from 300 to 8000 g/mol.

6. Compositions according to Claim 5, **characterized in that** the polyether polyols have a polydispersity (PD = M_{w}/Mₙ) in the range from 1.0 to 1.5 and an OH functionality of greater than 1.9 and also an unsaturated end group content of not more than 0.02 milliequivalents per gram of polyol (meq/g) (method of determination: ASTM D2849-69).

7. Compositions according to Claim 5, **characterized in that** the polyurethanes (1) of component B) are prepared using monohydroxyl-functional polyalkylene oxide polyether alcohols which on average include 5 to 70 ethylene oxide units per molecule and 30 to 100 mol% of ethylene oxide units and 0 to 70 mol% of propylene oxide units based on the total amount of oxyalkylene units.

8. Compositions according to Claim 1, **characterized in that** the anionically hydrophilicized polyurethanes in the form of aqueous dispersions are obtainable by
E) isocyanate-functional prepolymers being produced from at least
E1) organic polyisocyanates
E2) polymeric polyols having number average molecular weights in the range from 400 to 8000 g/mol and OH functionalities in the range from 1.5 to 6 and
E3) optionally hydroxyl-functional compounds having molecular weights in the range from 62 to 399 g/mol and
E4) optionally isocyanate-reactive, anionic or potentially anionic and/or optionally nonionic hydrophilicizing agents,
F) their free NCO groups then being wholly or partly reacted
F1) optionally with amino-functional compounds having molecular weights in the range from 32 to 400 g/mol and/or
F2) with isocyanate-reactive, preferably amino-functional, anionic or potentially anionic hydrophilicizing agents
by chain extension, and the prepolymers being dispersed in water before, during or after step F), any potentially ionic groups present being converted into the ionic form by partial or complete reaction with a neutralizing agent.

9. Process for producing polymer foams, wherein a composition according to any one of Claims 1 to 8 is foamed and dried.

10. Foams obtainable by following a process according to Claim 9.

11. Wound contact materials obtainable using compositions according to any one of Claims 1 to 8.

## Revendications

1. Compositions pouvant être obtenues par mélange au moins de polyuréthanes (I) présentant une teneur en groupes isocyanate libres de maximum 1,0% en poids et une teneur en unités d'oxyde d'éthylène (poids moléculaire = 44 g/mole), incorporées via des alcools monofonctionnels B) disposées dans des chaînes polyéther, de 10 à 95% en poids, qui ont été préparés par transformation de
A) prépolymères de polyisocyanate présentant une fonctionnalité NCO (moyenne) supérieure ou égale à 1, avec
B) 10 à 100% en équivalent, par rapport aux groupes isocyanate de A), d'un composant alcool monovalent, comprenant au moins un polyétheralcool monovalent présentant un poids moléculaire numérique moyen de 150 à 5000 g/mole, présentant une teneur en unités d'oxyéthylène de 30 à 100% en mole par rapport à la teneur totale en unités d'oxyalkylène du polyétheralcool monovalent,
C) 0 à 20% en équivalent, par rapport aux groupes isocyanate de A), d'un composant alcool monovalent, comprenant des alcools monovalents différents des composés du composant B) présentant un poids moléculaire numérique moyen de 32 à 5000 g/mole,
D) 0 à 80% en équivalent, par rapport aux groupes isocyanate de A), de composants au moins difonctionnels constitutifs dans le sens de la réaction d'addition de NCO présentant un poids moléculaire numérique moyen de 32 à 10 000 g/mole,
avec formation d'uréthane et le cas échéant d'urée, la réaction des groupes NCO le cas échéant en excès étant complétée par des réactions secondaires ayant lieu simultanément ou consécutivement jusqu'à une teneur résiduelle d'au maximum 1,0% en poids
et de polymères (II) pouvant être moussés
**caractérisées en ce qu'**on utilise, comme polymères (II) pouvant être moussés des polyuréthanes anioniquement hydrophilisés sous forme de dispersions aqueuses.

2. Compositions selon la revendication 1, **caractérisées en ce que** dans les polyuréthanes (I), la teneur en unités d'oxyde d'éthylène (poids moléculaire = 44 g/mole) est de 45 à 55% en poids par rapport au polyuréthane.

3. Compositions selon la revendication 1 ou 2, **caractérisées en ce que** la fonctionnalité NCO moyenne des prépolymères de polyisocyanate du composant A) vaut 1,8 à 2,2.

4. Compositions selon l'une quelconque des revendications 1 à 3, **caractérisées en ce qu'**on utilise, pour la préparation des composés du composant A), des polyols présentant des fonctionnalités OH de 1,8 à 2,5.

5. Compositions selon la revendication 4, **caractérisées en ce qu'**on utilise comme polyols, lors de la constitution des composés du composant A), des polyétherpolyols présentant des poids moléculaires numériques moyens Mₙ de 300 à 8000 g/mole.

6. Compositions selon la revendication 5, **caractérisées en ce que** les polyétherpolyols présentent une polydispersité (PD = M_{w}/Mₙ) de 1,0 à 1,5 et une fonctionnalité OH supérieure à 1,9 ainsi qu'une teneur en groupes terminaux insaturés inférieure ou égale à 0,02 milliéquivalent par gramme de polyol (méquiv/g) (procédé de détermination ASTM D2849-69).

7. Compositions selon la revendication 5, **caractérisées en ce que** lors de la préparation des polyuréthanes (I), on utilise dans le composant B) des poly(oxyde d'alkylène)-polyétheralcools à fonctionnalité monohydroxy, qui présentent en moyenne statistique 5 à 70 unités d'oxyde d'éthylène par molécule et 30 à 100% en mole d'unités d'oxyde d'éthylène et 0 à 70% en mole d'unités d'oxyde de propylène par rapport à la quantité totale d'unités d'oxyalkylène.

8. Compositions selon la revendication 1, **caractérisées en ce que** les polyuréthanes hydrophilisés par voie anionique (I) peuvent être obtenus sous forme de dispersions aqueuses **en ce que**
E) des prépolymères à fonctionnalité isocyanate sont préparés au moins à partir
E1) de polyisocyanates organiques
E2) de polyols polymères présentant des poids moléculaires numériques moyens de 400 à 8000 g/mole et des fonctionnalités OH de 1,5 à 6 et
E3) le cas échéant de composés à fonctionnalité hydroxy présentant des poids moléculaires de 62 à 399 g/mole et
E4) le cas échéant d'agents d'hydrophilisation réactifs avec isocyanate, anioniques ou potentiellement anioniques et/ou le cas échéant non ioniques,
F) dont les groupes NCO libres sont alors transformés totalement ou partiellement
F1) le cas échéant avec des composés à fonctionnalité amino présentant des poids moléculaires de 32 à 400 g/mole et/ou
F2) des agents d'hydrophilisation réactifs avec isocyanate, de préférence à fonctionnalité amino, anioniques ou potentiellement anioniques
avec allongement de chaîne et les prépolymères sont dispersés avant, pendant ou après l'étape F) dans l'eau, les groupes potentiellement ioniques le cas échéant contenus étant transformés en forme ionique par une transformation partielle ou totale avec un agent de neutralisation.

9. Procédé pour la préparation de mousses polymères, dans lequel une composition selon l'une quelconque des revendications 1 à 8 est moussée et séchée.

10. Mousses pouvant être obtenues selon un procédé selon la revendication 9.

11. Pansements, pouvant être obtenus en utilisant des compositions selon l'une quelconque des revendications 1 à 8.
